# FASCICULE DE BREVET EUROPEEN

(11) **EP 1 474 374 B1**
(45) Date de publication et mention de la délivrance du brevet: **14.05.2008**
(21) Numéro de dépôt: 03739531.6
(22) Date de dépôt: 12.02.2003
(51) Int. Cl.: C07C 45/78, C07C 45/80, C07C 45/82, C07C 47/22, C07C 319/18

(54) **PROCEDE DE PURIFICATION DE L'ACROLEINE**
VERFAHREN ZUR REINIGUNG VON ACROLEIN
METHOD FOR THE PURIFICATION OF ACROLEIN

(30) Priorité: 12.02.2002 FR 0201686
(43) Date de publication de la demande: 10.11.2004
(73) Titulaire: Adisseo Ireland Ltd, Dublin 1 (IE)
(72) Inventeur: GROS, Georges, F-92160 Antony (FR); GARRAIT, Michel, F-69390 Charly (FR); REY, Patrick, F-69006 Lyon (FR)
(74) Mandataire: Guerre, Dominique
(86) Numéro de dépôt international: PCT/FR2003/000454
(87) Numéro de publication internationale: WO 2003/068721

(56) Documents cités:
- FR-A- 2 460 925
- FR-A- 2 735 989
- US-A- 5 352 837

## Description

Le domaine de la présente invention est celui de la fabrication de l'acroléine en tant que produit intermédiaire ou produit final. Il concerne, en particulier, la purification de l'acroléine d'une phase aqueuse d'acroléine. L'invention relève également du domaine de la fabrication de l'AMTP, c'est-à-dire de l'aldéhyde 3-(méthylthio)propionique.

L'acroléine est une matière première, dont une utilisation majeure à l'échelle industrielle est la synthèse de l'AMTP par réaction d'acroléine avec du méthylmercaptan.

Les procédés mettant en oeuvre la fabrication de l'acroléine sont bien connus. Ces procédés comprennent généralement une étape de réaction d'oxydation du propylène et/ou du propane. Un produit brut gazeux à base d'acroléine peut être ainsi obtenu. Ce produit brut se présente généralement sous la forme d'un mélange gazeux comprenant, de première part de l'acroléine en une proportion généralement supérieure à 10% en poids, de seconde part des gaz inertes ou incondensables (encore appelés "off-gas") tels que de l'azote, de l'oxygène, du monoxyde et du dioxyde de carbone, du propylène, du propane, de troisième part de l'eau, et de quatrième part, des sous-produits de réaction tels que des acides, des aldéhydes, des alcools, et d'autres composés. Des traitements ultérieurs sont donc nécessaires pour éliminer certains des composés du produit brut et pour isoler de l'acroléine purifiée.

Le produit brut à base d'acroléine subit généralement un premier traitement permettant d'éliminer des acides, tels que l'acide acrylique et l'acide acétique. Un deuxième traitement permet ensuite d'absorber l'acroléine dans de l'eau, à l'aide d'une colonne d'absorption au fond de laquelle est recueillie une solution aqueuse d'acroléine. Cette solution subit ensuite une étape de purification pour isoler de l'acroléine purifiée sous forme gazeuse, à l'aide d'une ou plusieurs colonnes de distillation.

Souvent la purification de l'acroléine requiert l'utilisation de deux colonnes de distillation. La première colonne facultative permet de désoxygéner la solution et élimine les impuretés légères. La deuxième colonne est, quant à elle, opérée de façon à obtenir en tête de ladite colonne, et en particulier en sortie du condenseur, de l'acroléine liquide en concentration azéotropique avec de l'eau.

Que ce soit avec une ou plusieurs colonnes de distillation, l'encrassement de ces colonnes constitue un problème souvent rencontré lors de la purification de l'acroléine. Cet encrassement se caractérise souvent par le dépôt d'une phase solide résultant de la polymérisation des restes d'acide acrylique, voire d'acroléine. Dans le cas particulier des procédés de purification mettant en oeuvre deux colonnes, ces encrassements ont généralement lieu au niveau de la deuxième colonne qui est, par ailleurs, souvent utilisée dans des conditions azéotropiques.

Ces dépôts engendrent une diminution progressive de l'efficacité de la distillation et conduisent à un bouchage partiel, voire complet, de la colonne de distillation. Ce problème oblige donc à arrêter fréquemment les installations de production de l'acroléine, afin d'effectuer les travaux de maintenance qui s'imposent pour éliminer les dépôts dans la colonne ou les colonnes de distillation. Ces arrêts génèrent ainsi des coûts importants et une perte conséquente de la capacité des ateliers de production.

La présente invention vise donc un procédé pour purifier de l'acroléine, en minimisant les encrassements de la colonne de distillation utilisée, et ceci de façon simple, peu coûteuse et plus sécuritaire.

Il a donc été trouvé qu'en opérant une distillation d'une solution aqueuse d'acroléine avec des conditions opératoires particulières, il était possible de palier aux inconvénients liés à l'encrassement.

La présente invention a donc pour objet un procédé continu de purification d'acroléine dans lequel :
- on introduit une solution aqueuse d'acroléine dans une colonne de distillation équipée à sa base d'au moins un bouilleur et à sa tête d'au moins un condenseur,
- or soutire à la base de la colonne de distillation un mélange liquide comprenant de l'eau,
- on soutire en tête de la colonne de distillation un mélange gazeux comprenant de l'acroléine,
- on refroidit, dans le condenseur, le mélange gazeux soutiré en tête de colonne de distillation, à une température permettant d'obtenir d'une part, un condensat aqueux, et d'autre part un mélange gazeux riche en acroléine, et
- on soutire ledit mélange gazeux
caractérisé en ce que la distillation est déterminée pour obtenir à la base de la colonne un mélange liquide, non azéotropique, comprenant essentiellement de l'eau, et la condensation est déterminée pour obtenir un condensat aqueux substantiellement appauvri en acroléine et un mélange gazeux substantiellement enrichi en acroléine.

Préférentiellement, le mélange gazeux obtenu en tête de colonne comprend, en volume, entre 30 % et 70 %, et préférentiellement entre 40 % et 60 % d'eau.

Grâce aux choix opératoires précités, on effectue la distillation à des températures demeurant limitées, puisqu'en particulier on accepte en tête de colonne l'obtention d'un mélange gazeux contenant une proportion non négligeable d'eau. La séparation de l'eau est ensuite obtenue par des conditions particulières de refroidissement dans le condenseur, compensant en quelque sorte la séparation limitée de l'acroléine dans la colonne de distillation.

La présente invention a également pour objet un procédé de fabrication de l'AMTP, c'est-à-dire de l'aldéhyde 3-(méthylthio)propionique.

Les procédés de fabrication de l'AMTP de l'art antérieur impliquent généralement une réaction chimique de l'acroléine liquide avec du méthylmercaptan en présence généralement de catalyseur. Ces procédés nécessitent souvent un ou plusieurs stockages intermédiaires d'acroléine liquide. Ceci peut présenter un problème de sécurité lié aux caractéristiques de l'acroléine, en particulier, sa polymérisation, sa forte toxicité et son inflammabilité.

Pour répondre à ce problème de sécurité, d'autres procédés ont été développés par exemple dans US 422 5 516, US 431 9 047, et US 5 352 837.

Ils décrivent une purification partielle du mélange gazeux brut contenant l'acroléine, par exemple par deux refroidissements successifs, pour éliminer en particulier les acides. Le flux gazeux ainsi purifié ne contenant qu'une quantité limitée d'acroléine diluée dans les "off-gas" de la réaction, est mis en réaction avec le MSH, dans un réacteur par contact gaz/liquide.

Dans ce cas, les "off-gas" sont éliminés postérieurement à la réaction entre l'acroléine et le MSH; ils entraînent une perte substantielle d'acroléine et d'AMTP par "stripping" ou élution. De plus, les "off-gas" contenant ces fractions d'acroléine et AMTP nécessitent des traitements spécifiques et onéreux pour supprimer les odeurs et respecter la législation sur les rejets.

Pour répondre d'une part, au problème de sécurité, lié au stockage d'acroléine liquide et d'autre part, pour éviter des pertes de rendement par "stripping" de l'AMTP et le traitement onéreux de l'effluent gazeux, il a été trouvé qu'il était intéressant de mettre en oeuvre le procédé de purification de l'acroléine décrit précédemment et de fabriquer de l'AMTP en faisant réagir l'acroléine gazeuse ainsi purifiée obtenue par ledit procédé, directement avec du méthylmercaptan, liquide ou gazeux. Ainsi, la réaction de fabrication de l'AMTP peut être mise en oeuvre directement à partir de l'acroléine purifiée gazeuse et dépourvue de gaz dits "incondensables". Ce mode de fonctionnement permet ainsi d'éliminer tout stockage intermédiaire d'acroléine liquide, résoud ainsi le problème de sécurité évoqué précédemment ainsi que les pertes d'AMTP et d'acroléine.

La présente invention vise donc un procédé continu, ainsi pour la fabrication de l'AMTP comprenant une étape de purification de l'acroléine selon le procédé de purification de l'acroléine décrit ci-dessus.

La présente invention concerne en particulier un procédé continu de fabrication de l'AMTP, caractérisé en ce que :
(a) on réalise une oxydation en phase vapeur de propylène à l'aide d'un catalyseur, de façon à obtenir un produit brut à base d'acroléine,
(b) on élimine des acides contenus dans le produit brut obtenu à l'étape précédente,
(c) on absorbe avec de l'eau le produit obtenu à l'étape précédente, de façon à obtenir une solution aqueuse d'acroléine,
(d) on purifie, *par distillation,* ladite solution de façon à obtenir de l'acroléine purifiée gazeuse et,
(e) on fait réagir l'acroléine gazeuse purifiée, obtenue à l'étape précédente avec du MSH, c'est-à-dire un méthylmercaptan, de façon à obtenir l'AMTP,
*procédé dans lequel en outre:*
*l'étape (d) estt effectuée dans une colonne de distillation, à la tête de laquelle un mélange gazeux, comprenant essentiellement de l'acroléine et de l'eau, est soutiré et est refroidi pour obtenir un mélange gazeux riche en acroléine et duquel l'acroléine gazeuse purifiée est soutirée,*
et on sépare les gaz dits incondensables, originellement contenus dans le produit brut résultant de l'étape (a) d'oxydation, avant l'étape (e).

Préférentiellement, la séparation des gaz dits incondensables est effectuée avant l'étape de purification (d), en particulier pendant l'étape (b) et/ou l'étape (c), par exemple pendant l'étape (c).

Les gaz dits incondensables sont, ou recyclés vers l'étape (a) d'oxydation, ou évacués du procédé, et par exemple incinérés à une température relativement basse (900° C par exemple), s'agissant des rejets carbonés et non de rejets soufrés.

L'avantage déterminant d'une séparation des gaz dits incondensables, en amont de la synthèse de l'AMTP, est que lesdits gaz ne contiennent aucun composé soufré, généré par le MSH, et/ou l'AMTP, et peuvent donc être recyclés vers l'oxydation du propylène, en mélange avec ce dernier, sans risquer l'empoisonnement du catalyseur d'oxydation avec des composés soufrés.

La séparation des gaz dits incondensables, en amont de la purification de l'acroléine, permet aussi d'éliminer un ballast gazeux, normalement en proportion volumique relativement importante, pendant tout le procédé conduisant à l'AMTP.

Grâce à cette séparation amont des gaz dits incondensables, il n'y a pas lieu d'y procéder pendant ou postérieurement à la synthèse de l'AMTP, par réaction entre l'acroléine et le MSH, ce qui évite d'avoir à éliminer des composés soufrés avant rejet, par incinération des "off gas" à une température relativement haute (1200° C par exemple).

En cas de recyclage des gaz incondensables vers l'oxydation, le rendement en AMTP, exprimé par rapport à la quantité molaire entrante de propylène, se trouve substantiellement amélioré.

La présente invention concerne encore un procédé continu de fabrication de l'AMTF, caractérisé en ce que :
(a) on réalise une oxydation en phase vapeur de propylène à l'a de d'un catalyseur, de façon à obtenir un produit brut à base d'acroléine,
(b) on élimine des acides contenus dans le produit brut obtenu à l'étape précédente,
(c) on absorbe avec de l'eau le produit obtenu à l'étape précédente, de façon à obtenir une solution aqueuse d'acroléine, et séparée des gaz dits incondensables,
(d) on purifie ladite solution *par distillation* de façon à obtenir de l'acroléine purifiée gazeuse, *cette purification étant effectuée dans une colonne de distillation, à la tête de laquelle un mélange gazeux* (6), *comprenant essentiellement de l'acroléine et de l'eau, est soutiré et est refroidi pour obtenir un mélange gazeux (11) riche en acroléine, duquel l'acroléine gazeuse (12) purifiée est soutirée,* et,
(e) on fait réagir l'acroléine gazeuse purifiée, obtenue à l'étape précédente directement avec du MSH, liquide ou gazeux, c'est-à-dire un méthylmercaptan, de façon à obtenir l'AMTP.

Préférentiellement, l'étape (e) est effectuée entre du MSH liquide et de l'acroléine purifiée maintenue en phase gazeuse.

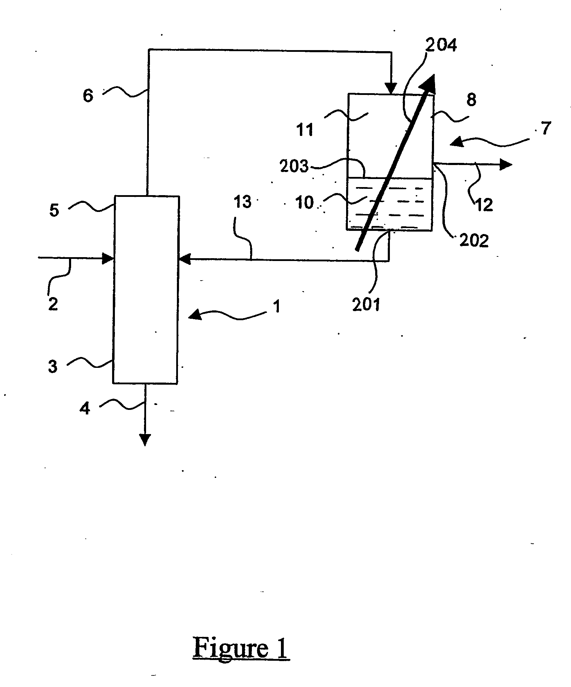

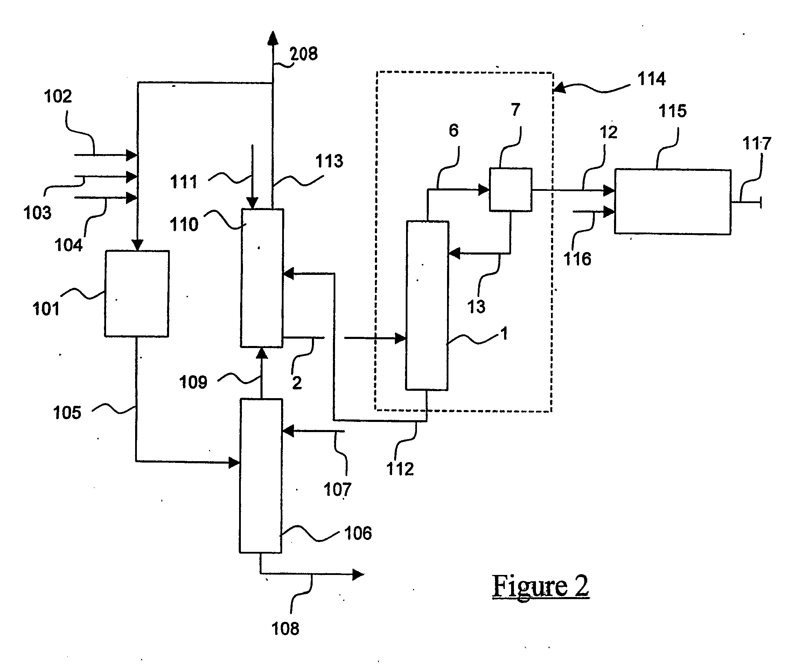

Les figures 1 et 2 représentent, de manière non limitative, un dispositif de purification de l'acroléine, ainsi qu'une installation pour la fabrication de l'AMTP mettant en oeuvre le procédé de l'invention.
La figure 1 illustre schématiquement un dispositif de purification de l'acroléine selon l'invention.
La figure 2 illustre schématiquement une installation de fabrication de L'AMTP selon l'invention.

La présente invention est décrite de manière plus détaillée dans ce qui suit, par référence à la figure 1.

*Selon un procédé de fabrication de l'AMTP, avantageux, l'étape (d) de purification de la solution aqueuse (2) d'acroléine est effectuée selon le procédé suivant:*
- *on introduit la solution aqueuse d'acroléine dans une colonne de distillation (1) équipée à* sa *base d'au moins un bouilleur et à sa tête d'au moins un condenseur (7),*
- *on soutire (4) à la base de la colonne de distillation un mélange liquide comprenant essentiellement de l'eau,*
- *on soutire (6) en tête (5) de la colonne de distillation un mélange gazeux comprenant essentiellement de l'acroléine et de l'eau,*
- *on refroidit, dans le condenseur, le mélange gazeux (6) soutiré en tête de colonne de distillation, à une température permettant d'obtenir d'une part, un condensât aqueux (13), et d'autre part, un mélange gazeux (12) riche en acroléine, et*
- *on soutire (12) le mélange gazeux riche en acroléine.*

Par gaz dits incondensables, ou "inertes", ou "off gas", on entend tout gaz ne pouvant pas être condensé, de manière relative, dans les conditions de purification selon le procédé de l'invention. Ces gaz ne peuvent généralement être condensés qu'à des températures bien au-dessous de 100°C. A titre d'exemple, ces gaz dits incondensables peuvent être de l'azote, du propylène, du propane ou de l'oxygène, qui sont généralement présents dans le flux gazeux résultant de la synthèse d'acroléine.

Les gaz dits incondensables ont été éliminés dans le procédé selon l'invention lors d'une étape préalable d'absorption de l'acroléine dans l'eau.

La solution 2 aqueuse d'acroléine, c'est-à-dire le mélange d'alimentation de la colonne de distillation, peut avoir une concentration en acroléine supérieure à 1%, et de toute façon inférieure ou égale à une concentration correspondant à la limite de solubilité de l'acroléine dans l'eau.

La solution 2 aqueuse d'acroléine a, de préférence, une concentration en acroléine inférieure ou égale à la limite de solubilité de l'acroléine dans l'eau, par exemple 5% en poids.

La colonne de distillation 1 est également équipée à sa base d'un bouilleur (non représenté) ayant pour fonction de vaporiser, au moins partiellement, la solution aqueuse obtenue en bas de colonne. Les conditions opératoires du bouilleur sont conventionnelles. La température dans le bouilleur peut être maintenue, dans le cas d'une colonne de distillation opérée à la pression atmosphérique, à une valeur allant de 100 à 130°C, de préférence de 100 à 120°C, spécialement de 102 à 110°C. L'homme de l'art saura adapter ces conditions de température selon que l'on travaille sous vide ou sous pression.

Selon l'invention, on soutire à la base de la colonne 1 de distillation un mélange 4 comprenant essentiellement de l'eau. Ce mélange soutiré peut néanmoins avoir une concentration en acroléine inférieure à 0, 1 % en poids, de préférence inférieure à 0,05% en poids, spécialement inférieure à 0,01% en poids.

Selon la présente invention, la solution 2 aqueuse d'acroléine est purifiée dans une colonne 1 de distillation, à la tête de laquelle est soutiré un mélange gazeux 6 comprenant essentiellement de l'acroléine et de l'eau. Ce mélange gazeux 6 est ensuite, dans un premier temps, refroidi par des moyens représentés schématiquement par la référence 204, de manière à obtenir un condensat 10 et une quantité substantielle d'un mélange gazeux 11 riche en acroléine. Dans un deuxième temps, l'acroléine purifiée 12 sous forme gazeuse est soutirée du mélange gazeux 11 riche en acroléine. Le refroidissement du mélange 6 soutiré en tête de colonne et le soutirage de l'acroléine purifiée 12 constituent deux étapes essentielles du procédé de l'invention, ces étapes pouvant intervenir simultanément ou consécutivement.

Selon l'un de ces aspects essentiels, le mélange 6 soutiré en tête de la colonne de distillation est donc refroidi dans un condenseur 7 de manière à obtenir le condensat 10 et une quantité substantielle d'un mélange gazeux 11 riche en acroléine.

Par "quantité substantielle", on entend que l'acroléine se retrouve, grâce aux conditions de refroidissement retenues, en grande partie dans le mélange gazeux 11, plutôt que dans le condensat 10. II peut être considéré que plus de 50%, de préférence plus de 70%, spécialement plus de 90% de la quantité massique d'acroléine initialement contenue dans le mélange gazeux 6 soutiré en tête de colonne 1 se retrouve, après refroidissement 204, purifié, sous forme gazeuse.

Un tel mélange gazeux riche en acroléine peut être obtenu par un choix judicieux de la température de refroidissement 204 du condenseur 7. Le choix de cette température doit être évidemment effectué en tenant compte de la valeur d'autres paramètres physiques tels que, par exemple, la pression.

Ainsi, selon un mode particulier de la présente invention, si la colonne 1 de distillation est maintenue à une pression P, la température dans le condenseur 7 doit être maintenue à une valeur T selon l'équation T>21,28*P+32,9, P étant exprimé en atmosphère. Par exemple si P est 1 atm, T est > 53°C, P est 2 atm, T est > à 75°C.

La colonne 1 est avantageusement maintenue à la pression atmosphérique, ce qui impose de maintenir une température dans le condenseur 7 à une valeur supérieure à 54°C, de préférence allant de 55 à 70°C, spécialement allant de 60 à 65°C.

Selon un autre aspect essentiel, l'acroléine purifiée 12 est soutirée du mélange gazeux 11 riche en acroléine, ce dernier ayant été obtenu par refroidissement 204 du mélange gazeux 6 soutiré en tête de colonne 1 de distillation.

Le verbe "soutirer" doit être compris de manière très générale. Selon l'invention, on entend par soutirer un produit d'un mélange, le fait de fractionner ou de prélever au moins une partie dudit mélange.

De préférence, l'acroléine purifiée 12 est obtenue par soutirage de la totalité du mélange gazeux 11 riche en acroléine. Dans ce cas, l'acroléine purifiée peut être isolée par simple séparation du mélange gazeux 11 riche en acroléine d'une part, et du condensat 10 d'autre part. L'acroléine purifiée 12 peut être ensuite soutirée par un conduit débouchant dans la partie supérieure du condenseur 7, contenant le mélange gazeux 11 riche en acroléine.

L'acroléine purifiée 12 se présente généralement sous la forme d'un mélange gazeux comprenant essentiellement de l'acroléine avec une faible teneur en eau. La purification porte principalement sur une réduction significative de la teneur en eau.

De manière avantageuse, à titre d'exemple, en ajustant la température de condensation, l'acroléine purifiée 12 peut avoir une concentration en acroléine allant de 86 à 95% en poids, de préférence de 88 à 94% en poids, spécialement de 90 à 93% en poids.

Selon un mode avantageux de la présente invention, on réintroduit 13, au moins partiellement, le condensat 10 dans la colonne de distillation. De préférence, le condensat est réintroduit totalement en tête de la colonne 1 de distillation.

Afin de réduire encore plus les encrassements, la solution d'acroléine 2 peut avantageusement subir une étape préalable de désoxygénation avant d'être alimentée dans la colonne 1 de distillation. Cette désoxygénation peut être effectuée par une mise sous vide de la solution d'acroléine 2.

De même, il peut être envisagé d'utiliser une colonne 1 de distillation ayant des plateaux à grandes perforations sans tube de rétrogradation, et ayant des parois chauffées de façon à éviter la condensation de liquide stagnant pouvant initier des points de polymérisation indésirables. Cependant cette possibilité peut se révéler coûteuse et inutile, vu les améliorations apportées par le procédé de la présente invention.

Les avantages du procédé de purification selon la présente invention sont de permettre :
- d'obtenir de l'acroléine en phase gazeuse 12 avec un degré de pureté élevé, sans nécessité d'arrêts de l'unité pour éliminer les encrassements de la colonne 1 et du condenseur 7, suite à la polymérisation de l'acide acrylique et/ou de l'acroléine,
- minimiser les besoins de frigories au niveau du condenseur 7,
- éviter la nécessité d'un stockage d'acroléine liquide et permettre donc de minimiser les encours de ce produit très toxique, inflammable et dangereux.

Par référence à la figure 2, un autre objet de la présente invention porte sur un procédé continu de fabrication de l'AMTP, c'est-à-dire l'aldéhyde 3-(méthylthio)propionique. Ce procédé est caractérisé en ce qu'il comprend une étape 114 de purification de l'acroléine selon le procédé décrit précédemment.

L'AMTP est un produit intermédiaire pour la fabrication de la méthionine ou du HMBA, c'est-à-dire de l'acide 2-hydroxy-4-(méthyl-thio) butanoïque. La méthionine est un acide aminé essentiel permettant de compenser les carences de l'alimentation animale. L'HMBA fournit une source de méthionine qui est couramment utilisée comme supplément de méthionine dans la formulation d'aliments pour animaux. L'AMTP est communément requis pour la fabrication de HMBA ou de méthionine.

Selon un mode préférentiel de l'invention, par référence à la figure 2, le procédé continu de fabrication de l'AMTP est caractérisé en ce que :
a) on réalise une oxydation 101 en phase vapeur du propylène 104 à l'aide d'un catalyseur, de façon à obtenir un produit brut gazeux 105 à base d'acroléine,
b) on élimine 106 des acides contenus dans le produit brut 105 obtenu à l'étape précédente,
c) on absorbe 110 avec de l'eau 111 le produit obtenu à l'étape précédente, de façon à obtenir une solution aqueuse 2 d'acroléine, substantiellement exempte d'acides, et séparée des gaz dits incondensables 113,
d) on purifie ladite solution 2 grâce au procédé 114 de purification de l'acroléine décrit précédemment, de façon à obtenir un flux 12 de l'acroléine purifiée gazeuse, et
e) on fait réagir en présence d'un catalyseur l'acroléine gazeuse 12 purifiée obtenue à l'étape précédente avec du MSH 116, gazeux ou liquide, c'est-à-dire un méthylmercaptan, de façon à obtenir l'AMTP 117.

Le produit brut 105 à base d'acroléine obtenu à la première étape (a) du procédé de fabrication de l'AMTP se présente généralement sous la forme d'un mélange gazeux comprenant de première part, de l'acroléine en une proportion supérieure à 5%, de préférence 10%, de deuxième part, des gaz incondensables tels que de l'azote, de l'oxygène, du monoxyde et du dioxyde de carbone, du propane, du propylène, de troisième part, de l'eau, et de quatrième part, des sous-produits de réaction tels que des acides, des aldéhydes, des alcools, et d'autres composés.

Ce produit brut 105 est ensuite traité, lors d'une deuxième étape (b) , de façon à éliminer, par n'importe quels moyens, des acides, tels que, par exemple, l'acide acrylique et l'acide acétique.

Le produit brut ainsi traité 109 peut être, selon l'étape (c) du procédé, mis en contact avec de l'eau refroidie 111 dans une colonne 110 d'absorption, afin de recueillir à la base de ladite colonne une solution aqueuse 2 d'acroléine, et en tête, un flux 113 d'''off-gas" ne contenant que des traces d'acroléine. Les gaz incondensables 113 peuvent être entièrement éliminés par le conduit 208, ou partiellement recyclés vers le procédé 101 d'oxydation du propylène 104.

La quatrième étape (d) du procédé de fabrication de l'AMTP consiste à purifier la solution aqueuse 2 d'acroléine selon le procédé de purification 114 décrit précédemment. Selon ce procédé de purification, on alimente, dans un premier temps, une colonne 1 de distillation avec une solution aqueuse 2 d'acroléine qui est dépourvue de gaz incondensables, ou "off-gas"

En ce qui concerne la cinquième étape (e) du procédé de fabrication de l'AMTP, c'est-à-dire la réaction de l'acroléine purifiée12 avec le MSH 116, il peut être envisagé d'utiliser de l'acroléine sous forme liquide ou gazeuse, en présence d'un catalyseur.

Selon un mode avantageux de la présente invention, la synthèse de l'AMTP est effectuée entre du MSH liquide 116 ou gazeux 116 et de l'acroléine 12 purifiée maintenue en phase gazeuse. L'avantage de ce mode réside, dans la simplification du procédé, et en particulier dans le fait d'éviter des stockages intermédiaires d'acroléine liquide qui seraient préjudiciables en termes de sécurité.

Un avantage de la présente invention est de permettre la synthèse de l'AMTP, en utilisant une source d'acroléine purifiée maintenue sous forme gazeuse.

Un autre avantage de ce procédé original qui utilise de l'acroléine sous forme de gaz, mais dépourvu de gaz incondensables, est d'éviter postérieurement à la synthèse de l'AMTP l'entraînement de composés soufrés et d'acroléine, qui nécessite un traitement onéreux et génère des pertes de rendement substantielles.

Un dispositif de purification de l'acroléine comprend:
- une conduite d'alimentation d'une solution aqueuse 2 d'acroléine dépourvue de gaz incondensables,
- une colonne 1 de distillation alimentée par la conduite d'alimentation,
- une conduite 6 de soutirage en tête de la colonne 1 de distillation,
- un condenseur 7 alimenté par la conduite de soutirage et muni d'un moyen 204 de refroidissement, afin de maintenir la température à des valeurs permettant d'obtenir un condensat 10 et une quantité substantielle d'un mélange gazeux 11 riche en acroléine, et
- une conduite 12 d'évacuation du condenseur 7 permettant d'isoler de l'acroléine purifiée dans le mélange gazeux riche en acroléine.

Le condenseur 7 est, de préférence, vertical pour permettre un écoulement par ruissellement le long de ces parois internes. Le condenseur 7 est, en particulier, muni :
- d'un orifice d'évacuation 201 du condensat 10 situé au-dessous d'un niveau 203 de condensat accumulé à la base du condenseur 7,
- d'un orifice 202 d'évacuation de l'acroléine purifiée sous forme gazeuse, situé au-dessus dudit niveau 203 de condensat, et
- de deux conduites 13 et 12 d'évacuation reliées à chacun desdits orifices.

La fabrication de l'AMTP comprend:
- un réacteur 101 permettant d'obtenir un produit brut gazeux 105 à base d'acroléine,
- un dispositif 106 d'élimination des acides, alimenté grâce à une conduite 105 d'alimentation en produit brut gazeux à base d'acroléine,
- un dispositif 110 d'absorption à l'eau de l'acroléine, alimenté grâce à une conduite 109 d'alimentation en produit brut gazeux à base d'acroléine dépourvu d'acides,
- un dispositif 114 pour la purification de l'acroléine alimenté grâce à une conduite 2 d'alimentation par une solution aqueuse d'acroléine, et
- un réacteur 115 de fabrication de l'AMTP alimenté grâce à une conduite 12 d'alimentation de l'acroléine purifiée et d'une conduite 116 d'alimentation en MSH,
caractérisée en ce que le dispositif 114 pour la purification de l'acroléine est conforme à celui décrit précédemment par référence à la figure 1.

De préférence, la conduite 12 d'alimentation du réacteur 115 de fabrication de l'AMTP est reliée directement à la conduite d'évacuation du condenseur 7 du procédé de purification.

La figure 1 illustre, de façon schématique et non limitative, une installation de purification de l'acroléine conforme à la présente invention.

Cette installation comprend une colonne de distillation 1 alimentée par une solution aqueuse d'acroléine par l'intermédiaire d'une conduite d'alimentation 2. La colonne de distillation 1 comporte une base 3 équipée d'un bouilleur non représenté sur la figure. Au niveau de cette base 3, un mélange comprenant essentiellement de l'eau est soutiré par l'intermédiaire d'une conduite de soutirage 4. La colonne de distillation comporte une tête 5 qui est reliée à une conduite de soutirage 6.

L'installation représentée à la figure 1 comprend également un condenseur 7, ce dernier étant alimenté par l'intermédiaire de la conduite de soutirage 6 disposée en tête 5 de la colonne de distillation 1. Le condenseur 7 est représenté schématiquement par une enceinte 8 et par une conduite 204 (représentée par une flèche) destinée à la circulation d'un fluide réfrigérant. L'enceinte 8 du condenseur 7 contient dans sa partie inférieure le condensat 10 et dans sa partie supérieure le mélange gazeux riche en acroléine 11. Le condenseur 7 est également relié à une conduite d'évacuation 12 de l'acroléine purifiée sous forme gazeuse. Cette conduite 12 débouche à l'intérieur de la partie supérieure de l'enceinte 8 contenant le mélange gazeux riche en acroléine.

L'installation de purification représentée à la figure 1 comporte également une conduite 13 permettant le recyclage dans la colonne de distillation du condensat 10 accumulé au fond de l'enceinte 8 du condenseur 7.
La figure 2 illustre de façon schématique et non limitative une installation pour la fabrication de l'AMTP conforme à la présente invention.

L'installation comporte un réacteur de fabrication de l'acroléine brute 101 alimenté par du propylène ou du propane, de l'air et de l'eau, par l'intermédiaire de conduites représentées respectivement sous les références 102, 103, 104. Un produit brut gazeux à base d'acroléine est obtenu dans le réacteur 101 par oxydation catalytique du propylène ou du propane par l'air en présence d'eau. Une conduite 105 reliée au réacteur 101 permet de transférer ledit produit brut vers un dispositif 106 d'élimination des acides.

Ce dispositif d'élimination des acides 106 est constitué par une colonne d'absorption à l'eau qui est alimentée, d'une part en produit brut gazeux à base d'acroléine par l'intermédiaire de la conduite 105, et d'autre part en eau par une conduite 107. Un effluent liquide comprenant des acides est évacué à la base de cette colonne par une conduite d'évacuation 108. En tête de la colonne d'absorption 106, un effluent gazeux comprenant l'acroléine et dépourvu d'acide est transféré par l'intermédiaire d'une conduite 109 vers un dispositif d'absorption de l'acroléine 110.

Ce dispositif d'absorption de l'acroléine 110 est également une colonne d'absorption à l'eau qui est alimentée en produit brut gazeux à base d'acroléine, dépourvu d'acide, par l'intermédiaire de la conduite 109 et en eau par l'intermédiaire des conduites 111 et 112. Les effluents gazeux obtenus en tête du dispositif 110 peuvent être partiellement recyclés dans le réacteur 101 de fabrication de l'acroléine par l'intermédiaire d'une conduite 113. Une solution d'acroléine comprenant essentiellement de l'acroléine et de l'eau est transférée à la base de la colonne d'absorption 110 grâce à la conduite d'alimentation 2 vers une installation 114 de purification de l'acroléine conforme à celle représentée et décrite par référence à la figure 1.

L'installation de purification 114 représentée à la figure 2 comprend donc, comme décrit précédemment :
- la conduite 2 d'alimentation en solution aqueuse d'acroléine,
- la colonne de distillation 1 reliée à ladite conduite 2,
- le condenseur 7 relié à la tête de la colonne de distillation 1 par la conduite de soutirage 6 et muni d'un moyen de refroidissement (non représenté), afin de maintenir la température à des valeurs permettant d'obtenir un condensat et un mélange gazeux riche en acroléine,
- une conduite d'évacuation 12 d'acroléine purifiée sous forme gazeuse, isolée dans le mélange gazeux riche en acroléine présent dans le condenseur 7, et
- une conduite 13 permettant le recyclage du condensât dans la colonne de distillation 1.

L'installation pour la fabrication de l'AMTP représentée à la figure 2 comprend également un réacteur 115 de fabrication de l'AMTP muni d'une conduite d'alimentation d'acroléine gazeuse purifiée, ladite conduite correspondant à la conduite d'évacuation 12. Le réacteur 115 est également alimenté par une conduite d'alimentation en MSH 116. L'AMTP produit est évacué par une conduite d'évacuation 117.

Les exemples suivants permettent de comprendre l'intérêt de la présente invention.

### Exemple 1

Cet exemple illustre un procédé de purification de l'acroléine de l'art antérieur pour lequel une seule colonne de distillation de 40 plateaux est utilisée. Cette colonne, maintenue à pression atmosphérique, était alimentée par une solution d'acroléine, comprenant 6 % en poids d'acroléine et 93,5 % en poids d'eau. Grâce à un bouilleur, la température à la base de la cette colonne était maintenue à une valeur de 110°C. En tête de cette colonne, un mélange azéotropique d'acroléine et d'eau fut soutiré et condensé totalement par l'intermédiaire d'un condenseur. Le mélange avait une concentration en acroléine de 95% en poids, les impuretés étant principalement constituées par de l'eau à hauteur de 3%, et de l'acétaldéhyde à hauteur de 1,5%. La production nominale en acroléine ainsi purifiée était de 70 tonnes par jour.

La colonne a fonctionné avec ces conditions opératoires pendant environ 3 à 4 semaines à son niveau nominal de production. Cette colonne a dû ensuite être arrêtée pour nettoyer les plateaux et les échangeurs de chaleur associés à cette colonne.

### Exemple 1 bis

Dans le but de minimiser les encrassements de la colonne utilisée à l'exemple 1, l'installation la comportant a été modifiée par l'adjonction d'un dégazage sous vide (20°C; 0,7 bar). Cette modification n'a pas permis de minimiser significativement les phénomènes d'encrassement observés précédemment.

### Exemple 2

Cet exemple illustre un procédé de purification de l'acroléine intégré dans une unité pilote pour la fabrication de l'AMTP selon la présente invention.

### Synthèse de l'acroléine

Un produit brut 105 à base d'acroléine est produit en sortie d'un réacteur 101 d'oxydation du propylène en acroléine en phase vapeur. Ce produit brut était constitué par un mélange gazeux ayant une température de 180°C et comprenant 63% en poids de gaz incondensables (propane, azote, oxygène, propylène, CO, CO2), 21% en poids d'eau, 12% en poids d'acroléine, 2% en poids d'acide acrylique et 2% d'autres composés.

### Absorption des acides

Ce produit brut à base d'acroléine, en phase vapeur, était introduit 105 à raison de 20 kg/h au pied d'une colonne 106 d'absorption, équipée avec des plateaux à trous, maintenue à une pression de 121000 Pa. Un liquide refroidi 108 comportant des acides et 1,3 % en poids d'acroléine était soutiré au pied de la colonne et maintenu à une température de 70.3°C. Une phase gazeuse acide est soutirée en tête de colonne, et ensuite refroidie à 4°C.

### Absorption de l'acroléine

La phase gazeuse acide ainsi obtenue était ensuite introduite à la base d'une colonne 110 d'absorption à l'eau avec un débit de 16,2 kg/h. Dans cette colonne d'absorption circulait un courant d'eau 111 introduite à 4°C afin d'absorber l'acroléine. Les gaz incondensables évoqués précédemment étaient évacués 113 en tête de la colonne d'absorption, et en pied de colonne est obtenue une solution aqueuse 2 d'acroléine à 6% en poids.

### Purification de l'acroléine

La purification de la solution aqueuse d'acroléine était effectuée selon le procédé de purification de l'invention, (cf. figure 1) à l'aide d'une seule colonne de distillation 1 comportant un garnissage de type vrac. Cette colonne, maintenue à pression atmosphérique, était donc alimentée 2 par une solution aqueuse d'acroléine à 6% en poids d'acroléine. Grâce à un bouilleur, la température à la base de la colonne de distillation était maintenue à 105°C. La tête de la colonne était équipée d'un condenseur 7 afin de refroidir le mélange soutiré en tête de colonne à 60°C. A cette température, un condensat 9 et un mélange gazeux 12 riche en acroléine étaient obtenus. Le condensat était réintroduit 13 dans sa totalité en tête de colonne 1. De l'acroléine purifiée avec une pureté de 93% en poids (les 7% restant étant principalement de l'eau) était isolée 12 par prélèvement de la totalité du mélange gazeux 8 riche en acroléine.

Après cinq semaines de marche, aucun encrassement n'a été observé sur le garnissage de la colonne 1 de distillation.

### Synthèse de l'AMTP

Dans un réacteur 115 bouclé avec recirculation d'AMTP, on introduit l'acroléine 12 purifiée sous forme gazeuse, et une quantité stoechiométrique de MSH liquide, en présence de catalyseur.

Le rendement est pratiquement quantitatif. La présente d'une quantité d'eau apportée par l'acroléine ainsi purifiée ne présente aucune gène en comparaison à de l'acroléine provenant d'une distillation azéotropique.

II apparaît clairement que le procédé de purification selon la présente invention permet de diminuer considérablement les encrassements dans la colonne de distillation utilisée pour la purification de l'acroléine.

## Revendications

1. Procédé continu de fabrication de l'aldéhyde 3-(méthylthio)propionique (AMTP), **caractérisé en ce que** :
a) on réalise une oxydation (101) en phase vapeur du propylène à l'aide d'un catalyseur, de façon à obtenir un produit brut (105) à base d'acroléine,
b) on élimine (106) des acides contenus dans le produit brut (105) obtenu à l'étape précédente,
c) on absorbe (110) avec de l'eau le produit obtenu à l'étape précédente, de façon à obtenir une solution aqueuse (2) d'acroléine
d) on purifie ladite solution (2), *par distillation,* de façon à obtenir de l'acroléine purifiée gazeuse (12), et
e) on fait réagir (115) l'acroléine gazeuse purifiée obtenue à l'étape précédente avec du MSH, c'est-à-dire un méthylmercaptan, de façon à obtenir l'AMTP,
*l'étape (d) étant effectuée dans une colonne de distillation, à la tête de laquelle un mélange gazeux (6), comprenant essentiellement de l'acroléine et de l'eau, est soutiré et est refroidi pour obtenir un mélange gazeux (11) riche en acroléine et duquel l'acroléine (92) gazeuse purifiée est soutirée,*
et ***en ce qu'**on* sépare les gaz dits incondensables, originellement contenus dans le produit brut (105) résultant de l'étape (a) d'oxydation, avant l'étape (e)

2. Procédé selon la revendication 1, **caractérisé en ce que** la séparation des gaz dits incondensables est effectuée avant l'étape de purification (d).

3. Procédé selon la revendication 2, **caractérisé en ce que** la séparation des gaz dits incondensables est effectuée pendant l'étape (b) et/ou l'étape (c).

4. Procédé selon la revendication 3, **caractérisé en ce que** la séparation des gaz dits incondensables est effectuée pendant l'étape (c).

5. Procédé selon la revendication 1, **caractérisé en ce que** les gaz dits incondensables, sont recyclés vers l'étape (a) d'oxydation.

6. Procédé selon la revendication 1, **caractérisé en ce que** les gaz dits incondensables sont évacués et incinérés.

7. Procédé continu de fabrication de l'AMTP selon la revendication 1 **caractérisé en ce que** :
a) on réalise une oxydation (101) en phase vapeur du propylène à l'aide d'un catalyseur, de façon à obtenir un produit brut (105) à base d'acroléine,
b) on élimine (106) des acides contenus dans le produit brut (105) obtenu à l'étape précédente,
c) on absorbe (110) avec de l'eau le produit obtenu à l'étape précédente, de façon à obtenir une solution aqueuse (2) d'acroléine et séparée des gaz dits incondensables,
d) on purifie ladite solution (2) *par distillation,* de façon à obtenir de l'acroléine purifiée gazeuse, *cette purification étant effectuée dans une colonne de distillation, à la tête de laquelle un mélange gazeux (6), comprenant essentiellement de l'acroléine et de l'eau, est soutiré et est refroidi pour obtenir un mélange gazeux (11) riche en acroléine, duquel l'acroléine gazeuse (12) purifiée est soutirée,* et
e) on fait réagir (115) l'acroléine gazeuse purifiée obtenue à l'étape précédente directement avec du MSH, c'est-à-dire du méthylmercaptan, de façon à obtenir l'AMTP.

8. Procédé selon la revendication 1 ou 7, **caractérisé en ce que** l'étape (e) est effectuée entre du MSH et de l'acroléine maintenue en phase gazeuse.

9. Procédé selon la revendication 1 ou 7, **caractérisé en ce que** l'étape (d) de purification de la solution aqueuse (2) d'acroléine est effectuée selon le procédé suivant :
- on introduit la solution aqueuse d'acroléine dans une colonne de distillation (1) équipée à sa base d'au moins un bouilleur et à sa tête d'au moins un condenseur (7),
- on soutire (4) à la base de la colonne de distillation un mélange liquide comprenant essentiellement de l'eau,
- on soutire (6) en tête (5) de la colonne de distillation un mélange gazeux comprenant essentiellement de l'acroléine et de l'eau,
- on refroidit, dans le condenseur, le mélange gazeux (6) soutiré en tête de colonne de distillation, à une température permettant d'obtenir d'une part, un condensât aqueux (13), et d'autre part, un mélange gazeux (12) riche en acroléine, et
- on soutire (12) le mélange gazeux riche en acroléine.

10. Procédé selon la revendication 9, **caractérisé en ce que** la solution aqueuse (2) d acroléine a une concentration en acroléine inférieure ou égale à la limite de solubilité de l'acroléine dans l'eau.

11. Procédé selon la revendication 9, **caractérisé en ce que** la colonne de distillation (1) est maintenue à une pression P et, **en ce que** la température dans le condenseur (7) est maintenue à une valeur T selon l'équation T>21,28*P+32,9.

12. Procédé selon la revendication 11, **caractérisé en ce que** la colonne (1) est maintenue à la pression atmosphérique et la température dans le condenseur est maintenue à une valeur supérieure à 54°C, de préférence allant de 55 à 70°C, spécialement allant de 60 à 65°C.

13. Procédé selon la revendication 1, **caractérisé en ce que** le mélange gazeux (2) riche en acroléine a un concentration en acroléine allant de 86 à 95% en poids, de préférence de 88 à 94 % en poids, spécialement de 90 à 93% en poids.

14. Procédé selon la revendication 1, **caractérisé en ce que** on réintroduit, au moins partiellement, le condensat (13) dans la colonne de distillation (1).

15. Procédé selon la revendication 14, **caractérisé en ce que** le condensat (13) est réintroduit totalement en tête de la colonne de distillation (1).

16. Procédé de purification de l'acroléine selon la revendication 9:
- on introduit (2) une solution aqueuse d'acroléine dans une colonne (1) de distillation équipée à sa base d'au moins un bouilleur et à sa tête d'au moins un condenseur (7),
- on soutire (4) à la base de la colonne de distillation un mélange liquide comprenant de l'eau,
- on soutire (6) en tête de la colonne de distillation un mélange gazeux comprenant de l'acroléine,
- on refroidit, dans le condenseur, le mélange gazeux (6) soutiré en tête de colonne de distillation, à une température permettant d'obtenir d'une part, un condensat aqueux (13), et d'autre part un mélange gazeux (12) riche en acroléine, et
- on soutire ledit mélange gazeux
**caractérisé en ce que** la distillation (1) est déterminée pour obtenir à la base de la colonne (1) un mélange liquide, non azéotropique, comprenant essentiellement de l'eau, et la condensation (7) est déterminée pour obtenir un condensat aqueux (13) substantiellement appauvri en acroléine et un mélange gazeux (12) substantiellement enrichi en acroléine.

17. Procédé selon la revendication 16, **caractérisé en ce que** *le mélange gazeux* obtenu en tête de colonne comprend, en volume, entre 30 % et 70 % et préférentiellement entre 40% et 60 % d'eau.

## Claims

1. Continuous process for the manufacture of 3-(methylthio)propionaldehyde (MTPA), **characterized in that**:
(a) a vapour-phase oxidation (101) of propylene is carried out using a catalyst, so as to obtain a crude acrolein-based product (105),
(b) acids present in the crude product (105) obtained in the preceding stage are removed (106),
(c) the product obtained in the preceding stage is absorbed (110) with water, so as to obtain an aqueous acrolein solution (2),
(d) said solution (2) is purified, by distillation, so as to obtain purified gaseous acrolein (12), and
(e) the purified gaseous acrolein obtained in the preceding stage is reacted (115) with MSH, that is to say methyl mercaptan, so as to obtain MTPA,
stage (d) being carried out in a distillation column, at the top of which is withdrawn a gas mixture (6) essentially comprising acrolein and water, which is cooled in order to obtain an acrolein-rich gas mixture (11) from which purified gaseous acrolein (12) is withdrawn,
and **in that** the "noncondensable" gases originally present in the crude product (105) resulting from oxidation stage (a) are separated before stage (e).

2. Process according to Claim 1, **characterized in that** the separation of the "noncondensable" gases is carried out before purification stage (d).

3. Process according to Claim 2, **characterized in that** the separation of the "noncondensable" gases is carried out during stage (b) and/or stage (c).

4. Process according to Claim 3, **characterized in that** the separation of the "noncondensable" gases is carried out during stage (c).

5. Process according to Claim 1, **characterized in that** the "noncondensable" gases are recycled to oxidation stage (a).

6. Process according to Claim 1, **characterized in that** the "noncondensable" gases are discharged and incinerated.

7. Continuous process for the manufacture of MTPA according to Claim 1, **characterized in that**:
(a) a vapour-phase oxidation (101) of propylene is carried out using a catalyst, so as to obtain a crude acrolein-based product (105),
(b) acids present in the crude product (105) obtained in the preceding stage are removed (106),
(c) the product obtained in the preceding stage is absorbed (110) with water, so as to obtain an aqueous acrolein solution (2) separated from the "noncondensable" gases,
(d) said solution (2) is purified, by distillation, so as to obtain purified gaseous acrolein, this purification being carried out in a distillation column, at the top of which is withdrawn a gas mixture (6) essentially comprising acrolein and water, which is cooled in order to obtain an acrolein-rich gas mixture (11) from which purified gaseous acrolein (12) is withdrawn, and
(e) the purified gaseous acrolein obtained in the preceding stage is reacted (115) directly with MSH, that is to say methyl mercaptan, so as to obtain MTPA.

8. Process according to Claim 1 or 7, **characterized in that** stage (e) is carried out between MSH and acrolein maintained in the gas phase.

9. Process according to Claim 1 or 7, **characterized in that** stage (d) of purification of the aqueous acrolein solution (2) is carried out according to the following process:
- the aqueous acrolein solution is introduced into a distillation column (1) equipped at its base with at least one boiler and at its top with at least one condenser (7),
- a liquid mixture essentially comprising water is withdrawn (4) at the base of the distillation column,
- a gas mixture essentially comprising acrolein and water is withdrawn (6) at the top (5) of the distillation column,
- the gas mixture (6) withdrawn at the top of the distillation column is cooled, in the condenser, to a temperature which makes it possible to obtain, on the one hand, an aqueous condensate (13) and, on the other hand, an acrolein-rich gas mixture (12), and
- the acrolein-rich gas mixture is withdrawn (12).

10. Process according to Claim 9, **characterized in that** the aqueous acrolein solution (2) has a concentration of acrolein of less than or equal to the solubility limit of acrolein in water.

11. Process according to Claim 9, **characterized in that** the distillation column (1) is maintained at a pressure P and **in that** the temperature in the condenser (7) is maintained at a value T according to the equation T>21.28*P+32.9.

12. Process according to Claim 11, **characterized in that** the column (1) is maintained at atmospheric pressure and the temperature in the condenser is maintained at a value of greater than 54°C, preferably ranging from 55 to 70°C, especially ranging from 60 to 65°C.

13. Process according to Claim 1, **characterized in that** the acrolein-rich gas mixture (2) has an acrolein concentration ranging from 86 to 95% by weight, preferably from 88 to 94% by weight, especially from 90 to 93% by weight.

14. Process according to Claim 1, **characterized in that** the condensate (13) is at least partially reintroduced into the distillation column (1).

15. Process according to Claim 14, **characterized in that** all of the condensate (13) is reintroduced at the top of the distillation column (1).

16. Process for the purification of acrolein according to Claim 9, in which:
- an aqueous acrolein solution is introduced (2) into a distillation column (1) equipped at its base with at least one boiler and at its top with at least one condenser (7),
- a liquid mixture comprising water is withdrawn (4) at the base of the distillation column,
- a gas mixture comprising acrolein is withdrawn (6) at the top of the distillation column,
- the gas mixture (6) withdrawn at the top of the distillation column is cooled, in the condenser, to a temperature which makes it possible to obtain, on the one hand, an aqueous condensate (13) and, on the other hand, an acrolein-rich gas mixture (12), and
- said gas mixture is withdrawn,
**characterized in that** the distillation (1) is determined in order to obtain, at the base of the column (1), a nonazeotropic liquid mixture essentially comprising water and the condensation (7) is determined in order to obtain an aqueous condensate (13) substantially depleted in acrolein and a gas mixture (12) substantially enriched in acrolein.

17. Process according to Claim 16, **characterized in that** the gas mixture obtained at the column top comprises, by volume, between 30% and 70% and preferably between 40% and 60% of water.

## Patentansprüche

1. Kontinuierliches Verfahren zur Herstellung von 3-Methylthiopropionaldehyd (MTPA), **dadurch gekennzeichnet, dass**:
a) eine Oxidation (101) von Propylen in der Dampfphase mit Hilfe eines Katalysators durchgeführt wird, so dass ein Rohprodukt (105) auf Acroleinbasis erhalten wird,
b) Säuren entfernt werden (106), die in dem im vorhergehenden Schritt erhaltenen Rohprodukt (105) enthalten sind,
c) das im vorhergehenden Schritt erhaltene Produkt mit Wasser absorbiert wird (110), so dass eine wässrige Acroleinlösung (2) erhalten wird,
d) die Lösung (2) durch Destillation gereinigt wird, so dass gereinigtes, gasförmiges Acrolein (12) erhalten wird und
e) das im vorhergehenden Schritt erhaltene gereinigte, gasförmige Acrolein mit MSH, das heißt einem Methylmercaptan, umgesetzt wird (115), so dass MTPA erhalten wird,
wobei der Schritt (d) in einer Destillationskolonne durchgeführt wird, an deren Kopfende ein gasförmiges Gemisch (6), welches im Wesentlichen Acrolein und Wasser umfasst, abgezogen wird und abgekühlt wird, um ein gasförmiges Gemisch (11) zu erhalten, das reich an Acrolein ist und aus dem das gereinigte, gasförmige Acrolein (12) abgezogen wird,
und **dadurch**, dass die ursprünglich in dem aus dem Oxidationsschritt (a) hervorgegangenen Rohprodukt (105) enthaltenen nicht kondensierbaren Gase vor dem Schritt (e) abgetrennt werden.

2. Verfahren nach Anspruch 1, **dadurch gekennzeichnet, dass** das Abtrennen der nicht kondensierbaren Gase vor dem Schritt (d) erfolgt.

3. Verfahren nach Anspruch 2, **dadurch gekennzeichnet, dass** das Abtrennen der nicht kondensierbaren Gase während des Schritts (b) und/oder des Schritts (c) erfolgt.

4. Verfahren nach Anspruch 3, **dadurch gekennzeichnet, dass** das Abtrennen der nicht kondensierbaren Gase während des Schritts (c) erfolgt.

5. Verfahren nach Anspruch 1, **dadurch gekennzeichnet, dass** die nicht kondensierbaren Gase zum Oxidationsschritt (a) zurückgeführt werden.

6. Verfahren nach Anspruch 1, **dadurch gekennzeichnet, dass** die nicht kondensierbaren Gase abgeleitet und verbrannt werden.

7. Kontinuierliches Verfahren zur Herstellung von MTPA nach Anspruch 1, **dadurch gekennzeichnet, dass**:
a) eine Oxidation (101) von Propylen in der Dampfphase mit Hilfe eines Katalysators durchgeführt wird, so dass ein Rohprodukt (105) auf Acroleinbasis erhalten wird,
b) Säuren entfernt werden (106), die in dem im vorhergehenden Schritt erhaltenen Rohprodukt enthalten sind,
c) das im vorhergehenden Schritt erhaltene Produkt mit Wasser absorbiert wird (110), so dass eine wässrige und von nicht kondensierbaren Gasen abgetrennte Acroleinlösung (2) erhalten wird,
d) die Lösung (2) durch Destillation gereinigt wird, um gereinigtes, gasförmiges Acrolein zu erhalten, wobei die Reinigung in einer Destillationskolonne durchgeführt wird, an deren Kopfende ein gasförmiges Gemisch (6), welches im Wesentlichen Acrolein und Wasser umfasst, abgezogen wird und abgekühlt wird, so dass ein gasförmiges Gemisch (11) erhalten wird, das reich an Acrolein ist, aus dem das gereinigte, gasförmige Acrolein (12) abgezogen wird, und
e) das im verhergehenden Schritt erhaltene gereinigte, gasförmige Acrolein direkt mit MSH, das heißt mit Methylmercaptan, umgesetzt wird (115), so dass MTPA erhalten wird.

8. Verfahren nach Anspruch 1 oder 7, **dadurch gekennzeichnet, dass** der Schritt (e) zwischen MSH und Acrolein erfolgt, die in der Gasphase gehalten werden.

9. Verfahren nach Anspruch 1 oder 7, **dadurch gekennzeichnet, dass** der Schritt (d) der Reinigung der wässrigen Acroleinlösung (2) nach folgendem Verfahren erfolgt:
- die wässrige Acroleinlösung wird in eine Destillationskolonne (1) eingeführt, die an ihrem Fußende mit mindestens einem Brenner und an ihrem Kopfende mit mindestens einem Kondensator (7) versehen ist,
- am Fußende der Destillationskolonne wird ein flüssiges Gemisch abgezogen (4), das im Wesentlichen Wasser umfasst,
- am Kopfende (5) der Destillationskolonne wird ein gasförmiges Gemisch abgezogen (6), das im Wesentlichen Acrolein und Wasser umfasst,
- das am Kopfende der Destillationskolonne abgezogene gasförmige Gemisch (6) wird im Kondensator auf eine Temperatur abgekühlt, die es gestattet, einerseits ein wässriges Kondensat (13) und andererseits ein gasförmiges Gemisch (12) zu erhalten, das reich an Acrolein ist und
- das an Acrolein reiche, gasförmige Gemisch (12) wird abgezogen.

10. Verfahren nach Anspruch 9, **dadurch gekennzeichnet, dass** die wässrige Acroleinlösung (2) eine Konzentration an Acrolein aufweist, die kleiner oder gleich der Löslichkeitsgrenze von Acrolein in Wasser ist.

11. Verfahren nach Anspruch 9, **dadurch gekennzeichnet, dass** die Destillationskolonne (1) bei einem Druck P gehalten wird und **dadurch**, dass die Temperatur im Kondensator (7) bei einem Wert T gemäß der Gleichung T>21,28*P+32,9 gehalten wird.

12. Verfahren nach Anspruch 11, **dadurch gekennzeichnet, dass** die Destillationskolonne (1) bei atmosphärischem Druck gehalten wird und die Temperatur im Kondensator bei einem Wert oberhalb von 54 °C gehalten wird, vorzugsweise zwischen 55 und 70 °C, insbesondere zwischen 60 und 65 °C.

13. Verfahren nach Anspruch 1, **dadurch gekennzeichnet, dass** das an Acrolein reiche gasförmige Gemisch (2) eine Acroleinkonzentration zwischen 86 und 95 Gew.%, vorzugsweise zwischen 88 und 94 Gew.%, insbesondere zwischen 90 und 93 Gew.% aufweist.

14. Verfahren nach Anspruch 1, **dadurch gekennzeichnet, dass** das Kondensat (13) wenigstens teilweise in die Destillationskolonne (1) wieder eingeführt wird.

15. Verfahren nach Anspruch 14, **dadurch gekennzeichnet, dass** das Kondensat (13) vollständig in das Kopfende der Destillationskolonne (1) wieder eingeführt wird.

16. Verfahren zur Reinigung von Acrolein nach Anspruch 9, wobei
- eine wässrige Acroleinlösung in eine Destillationskolonne (1) eingeführt wird (2), die an ihrem Fußende mit mindestens einem Brenner und an ihrem Kopfende mit mindestens einem Kondensator (7) versehen ist,
- am Fußende der Destillationskolonne ein flüssiges Gemisch abgezogen wird (4), das Wasser umfasst,
- am Kopfende (5) der Destillationskolonne ein gasförmiges Gemisch abgezogen wird (6), das Acrolein umfasst,
- das am Kopfende der Destillationskolonne abgezogene gasförmige Gemisch (6) im Kondensator auf eine Temperatur abgekühlt wird, die es gestattet, einerseits ein wässriges Kondensat (13) und andererseits ein gasförmiges Gemisch (12) zu erhalten, das reich an Acrolein ist und
- das gasförmige Gemisch abgezogen wird,
**dadurch gekennzeichnet, dass** die Destillation so bemessen ist, dass am Fußende der Kolonne (1) ein nicht azeotropes, flüssiges Gemisch erhalten wird, das im Wesentlichen Wasser umfasst und die Kondensation (7) so bemessen ist, dass ein wässriges Kondensat (13) erhalten wird, das substantiell arm an Acrolein ist sowie ein gasförmiges Gemisch (12), das substantiell reich an Acrolein ist.

17. Verfahren nach Anspruch 16, **dadurch gekennzeichnet, dass** das am Kopfende der Kolonne erhaltene gasförmige Gemisch zwischen 30 und 70 Vol.% und vorzugsweise zwischen 40 und 60 Vol.% Wasser umfasst.
